# EUROPEAN PATENT SPECIFICATION

(11) **EP 2 049 558 B1**
(45) Date of publication and mention of the grant of the patent: **21.03.2012**
(21) Application number: 07793303.4
(22) Date of filing: 30.07.2007
(51) Int. Cl.: C07H 19/073

(54) **NAPHTHALENE 2-CARBOXYLATE DERIVATIVE USEFUL FOR SYNTHESIZING GEMCITABINE AND A METHOD FOR PREPARING THE SAME**
FÜR DIE SYNTHESE VON GEMCITABIN GEEIGNETES NAPHTHALIN-2-CARBOXYLATDERIVAT UND VERFAHREN ZU DESSEN HERSTELLUNG
DÉRIVÉ DE NAPHTHALÈNE 2-CARBOXYLATE UTILE POUR LA SYNTHÈSE DE GEMCITABINE ET PROCÉDÉ DE PRÉPARATION CORRESPONDANT

(30) Priority: 01.08.2006 KR 20060072786
(43) Date of publication of application: 22.04.2009
(73) Proprietor: Yuil Pharm Tech Co., Ltd., Chungcheongbuk-do 365-824 (KR); Chiro Genix Co., Ltd., Kyunggi-do 445-743 (KR); Kim, Kyu Wann, Gyeonggi-do 448-980 (KR); Kim, Kyoung Soo, Gyeonggi-do 443-737 (KR); Park, Young Won, Seoul 156-841 (KR); Park, Young Jun, Gyeonggi-do 443-470 (KR); Lee, Won Kyoung, Chungcheongbuk-do 360-182 (KR)
(72) Inventor: KIM, Kyu Wann, Yongin-si Gyeonggi-do 448-980 (KR); KIM, Kyoung Soo, Suwon-si Gyeonggi-do 443-737 (KR); PARK, Young Won, Dongjak-gu Seoul 156-841 (KR); PARK, Young Jun, Suwon-si Gyeonggi-do 443-736 (KR); LEE, Won Kyoung, Cheongju-si Chungcheongbuk-do 360-182 (KR)
(74) Representative: Schrell, Andreas
(86) International application number: PCT/KR2007/003648
(87) International publication number: WO 2008/016244

(56) References cited:
- WO-A2-2007/027564
- US-A- 6 001 994
- US-A- 6 013 790
- US-A1- 2004 142 857

## Description

### [Technical Field]

The present invention relates to naphthalene 2-carboxylate derivatives, which are useful for the synthesis of gemcitabine represented by Formula (II), and to a preparation method thereof.

Gemcitabine (2'-deoxy-2',2'-difluorocytidine), which is a well-known potent anticancer compound having a wide range of anticancer activities, is represented by Formula (II):

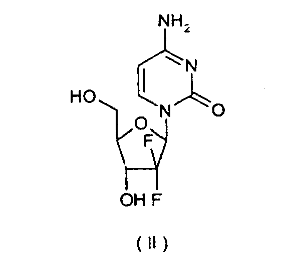

Pharmaceutical formulations containing this compound are used for the treatment of locally advanced or metastasized non-small cell lung cancer, locally advanced or metastasized pancreatic cancer, bladder cancer, advanced breast cancer and the like.

As shown in Formula (II), gemcitabine is an erythro enantiomer, in which the hydroxy group at the 3-position is oriented down. This erythro enantiomer is a naturally occurring configuration and shows excellent pharmaceutical effects compared to a threo enantiomer, in which the hydroxy group is oriented upward.

### '[Background Art]

In the preparation of gemcitabine, it is very important to effectively synthesize 1-oxoribose compounds, in which the 3-hydroxy group is oriented in the erythro configuration. For this reason, many efforts have been made to synthesize such intermediates.

Korean Patent Registration No. 22311 discloses the use of a compound represented by Formula (V) for the preparation of difluoro antiviral agents such as gemcitabine. As shown in Reaction Scheme 1, the compound represented by Formula (V) is prepared by allowing a compound of Formula (III) to react with Dowex 50W-X12 to prepare a lactone ring compound of Formula (IV'), and introducing a hydroxy protecting group into the compound (V). However, this method has a problem in that the compound of Formula (V) is isolated in a very low yield in the final step of the gemcitabine preparation process, because it exists in a 3:1 mixture of an erythro enantiomer and a threo enantiomer.

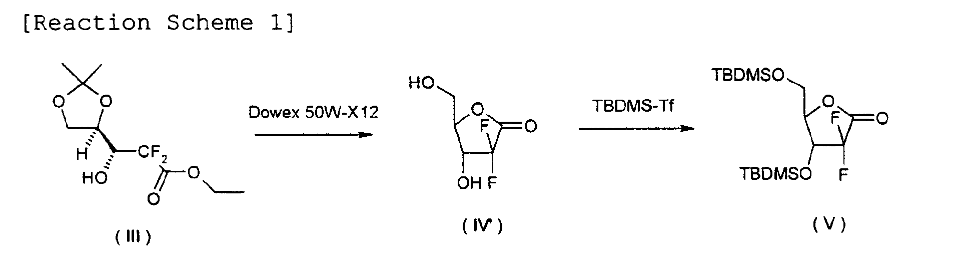

US Patent No. 4,526,988 discloses a method of preparing a compound of Formula (IV) by carrying out the separation of a 3:1 mixture of the following 3-R-hydroxy enantiomer and the 3-S-hydroxy enantiomer using column chromatography:

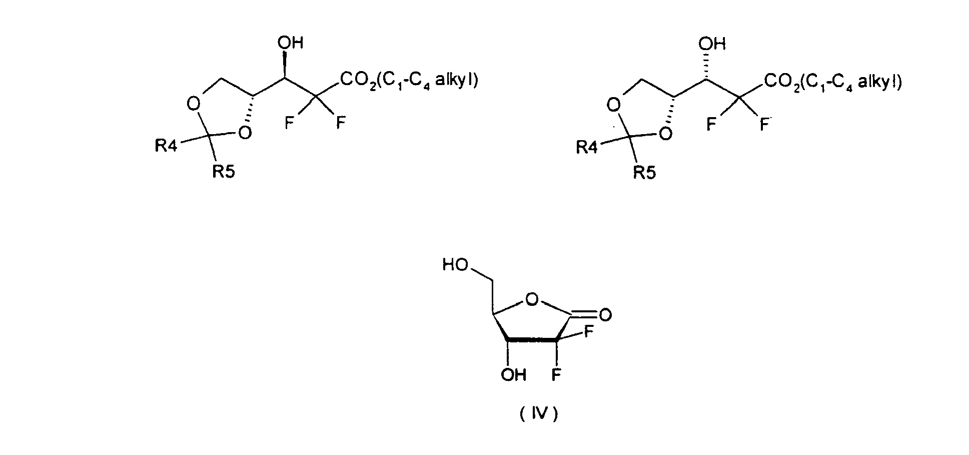

This method allows the preparation of the erythro compound of Formula (IV), but has problems in that it is required to use column chromatography, which is expensive and difficult to use *in situ.*

Korean Patent Registration No. 117181 discloses a method for preparing a compound represented by Formula (VI). As shown in Reaction Scheme 2 below, the compound represented by Formula (VI) is prepared by reacting a compound of Formula (III), which is a 3:1 mixture of 3R- and 3S-, with a strong acid such as sulfuric acid, to prepare a compound of Formula (IV'), protecting the hydroxy group of the compound of Formula (IV') with benzoyl to prepare a compound of Formula (VI'), which is a mixture of an erythro enantiomer and a threo enantiomer, and then separating an erythro enantiomer of Formula (VI) from the compound (VI'). According to this method, only the desired enantiomer can be selectively separated from the crystallizable compound of Formula (VI), prepared by introducing benzoyl derivatives, but the reaction yield is as low as about 23%. 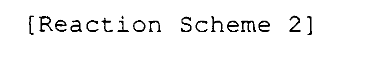 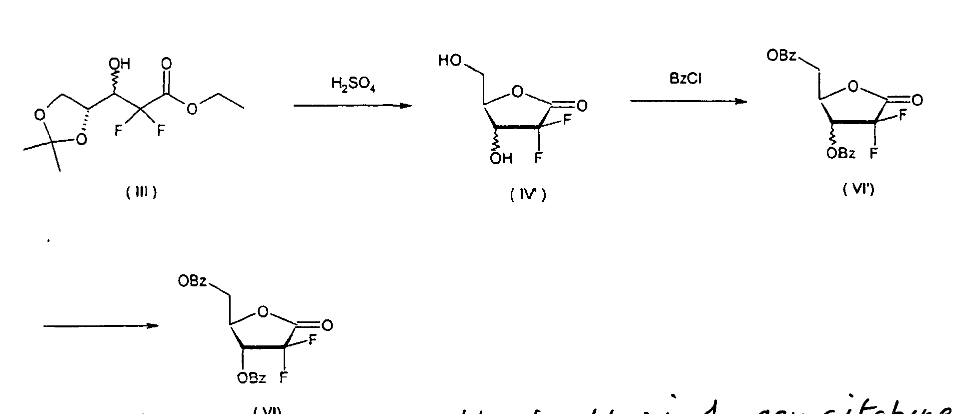 Also US 600 199 4 discloses the synthesis of gemcitabine via intermediate VI.

Korean Patent Laid-Open Publication No. 2006-0008056 discloses a method for preparing an erythro compound represented by Formula (IX). As shown in Reaction Scheme 3 below, the compound of Formula (IX) is prepared by protecting a compound of Formula (III) with biphenylcarbonyl to prepare a solid, 3R-carboxylate enantiomer of Formula (VIII), and then allowing the compound of Formula (VIII) to react with a strong acid. According to this method, the erythro compound of Formula (IX) is effectively prepared by selectively separating the 3R-carboxylate enantiomer from the compound of Formula (VIII) before carrying out the lactonization reaction. In this method, the reaction yield is greatly increased to 44%, because the desired enantiomer is effectively separated from the new intermediate of Formula (VIII), but there is a problem in that the number of reaction steps is greatly increased.

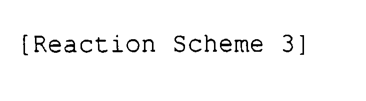

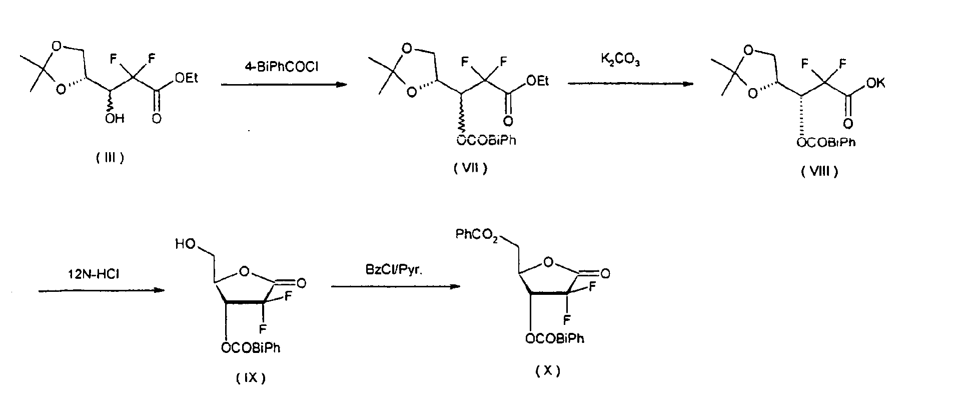

### [Disclosure]

### [Technical Problem]

Accordingly, the present inventors have conducted many studies to solve the above-mentioned problems occurring in the prior art and to develop a novel intermediate, from which gemcitabine can be more easily prepared. As a result, the present inventors have found that compounds of Formula I, obtained by protecting the 3- and 5-hydroxyl groups of 2-deoxy-2,2-difluoro-pentofuranose-1-ulose of Formula (IV') with naphthalene-2-carboxylate derivatives, are useful for the synthesis of gemcitabine.

That is to say, the present inventors have developed novel derivatives, from which the desired erythro enantiomer can be effectively separated, as well as a preparation method, in which the novel derivatives are prepared by introducing naphthalene-2-carboxylate derivatives, having high crystallinity, into the 3- and 5-hydroxy groups of 2-deoxy-2,2-difluoro-pentofuranose-1-ulose represented by Formula (IV').

Particularly, the present inventors have conducted studies focused on finding novel protecting groups having both the highest crystallinity and the highest selectivity, considering that the selective crystallization of the desired erythro enantiomer is the most difficult problem in the synthesis of gemcitabine represented by Formula (II).

As a result, the present inventors have developed a novel method, in which erythro compounds represented by Formula (I) can be prepared by allowing 2-deoxy-2,2-difluoro-pentofuranose-1-ulose of Formula (IV') with 2-naphthoyl halide derivatives of Formula (XI) in a reaction solvent in the presence of 2,6-lutidine and 4-dimethylaminopyridine.

Thus, according to the present invention, novel naphthalene-2-carboxylate derivatives of Formula 1, from which gemcitabine can be easily prepared using a simpler and easier process compared to the prior art, could be developed.

Accordingly, it is an object of the present invention to provide naphthalene-2-carboxylate derivatives of Formula (I), which are novel intermediates for preparing gemcitabine that is the key component of promising anticancer agents, as well as a novel preparation method thereof.

Another object of the present invention is to provide novel naphthalene-2-carboxylate derivatives of Formula (I), which can be used for the preparation of gemcitabine in an easier and more economical manner, as well as a novel preparation method thereof.

Still another object of the present invention is to provide novel naphthalene-2-carboxylate derivatives of Formula (I), which are suitable for preparing gemcitabine in large amounts, as well as a novel preparation method thereof.

These and other objects can be achieved according to the present invention as described below.

### [Technical Solution]

The present invention provides novel naphthalene-2-carboxylate derivatives represented by Formula (I) below, and a preparation method thereof: 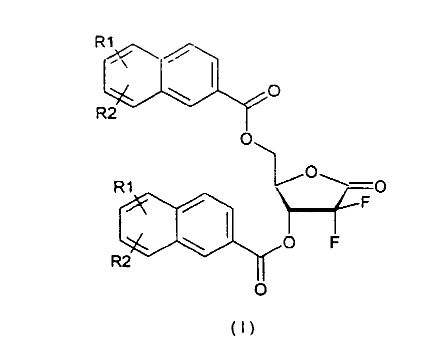 wherein R1 and R2 are each independently is hydrogen, methyl, chloro, fluoro, bromo, iodo, methoxy, ethoxy or nitro.

The novel naphthalene-2-carboxylate derivatives can be obtained by allowing 2-deoxy-2,2-difluoro-pentofuranose-l-ulose of Formula (IV') with 2-naphthoyl halide derivatives of Formula (XI) in a reaction solvent in the presence of 2,6-lutidine and 4-dimethylaminopyridine, thus preparing erythro compounds represented by Formula (I).

Thus, the present invention provides a novel method for the high-yield production of novel naphthalene-2-carboxylate derivatives of Formula (I), which are prepared by introducing 2-naphthoyl groups as described above and from which gemcitabine can be prepared using a simpler and easier process.

A preferred embodiment of the present invention is shown in Reaction Scheme 4 below. 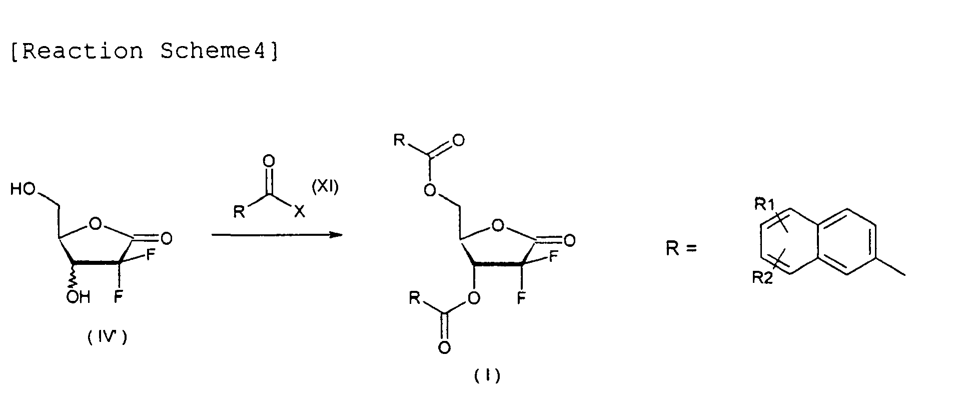 wherein R1 and R2 are each independently hydrogen, methyl, chloro, fluoro, bromo, iodo, methoxy, ethoxy or nitro, and X is chloride or bromide.

The naphthalene-2-carboxylate derivatives of Formula (I), obtained by protecting the 3- and 5-hydroxyl groups of 2-deoxy-2,2-difluoro-pentofuranose-1-ulose of Formula (IV') with naphthalene-2-carboxylate derivatives having high crystallinity, are novel compounds, and it is an object of the present invention to provide novel naphthalene-2-carboxylate derivatives useful for the synthesis of gemcitabine represented by Formula (I).

### [Best Mode]

Hereinafter, the present invention will be described in further detail.

According to the present invention, D-erythro-2-deoxy-2,2-difluoro-pentofuranose-1-ulose-3,5-di(naphthalene-2-carboxylate) derivatives represented by Formula (I) are prepared by allowing 2-deoxy-2,2-difluoro-pentofuranose-1-ulose of Formula (IV') to react with 2-napthoyl halide derivatives of Formula (XI) in a reaction solvent in the presence of 2,6-lutidine and 4-dimethylaminopyridine.

The 2-deoxy-2,2-difluoro-pentofuranose-1-ulose of Formula (IV') can be easily prepared according to the known method described in, for example, Korean Patent Registration No. 22311.

The 2-naphthoyl halide derivatives are compounds having 0-2 substituent groups introduced at position 5, 6, 7 or 8 of 2-naphthoyl halide, in which the substutient groups include methyl, chloro, fluoro, bromo, iodo, methoxy, ethoxy, nitro and the like, and the halide is chloride or bromide. The 2-naphthoyl halide derivatives are used in an amount of 2-2.5 equivalents, and preferably 2.2-2.4 equivalents, based on the 2-deoxy-2,2-difluoro-pentofuranose-l-ulose of Formula (IV'). Also, 2,6-luthidine as a base is used in an amount of 2.5-3.5 equivalents based on the 2-deoxy-2,2-difluoro-pentofuranose-1-ulose of Formula (IV'). In addition, 4-dimethylaminopyridine is used in an amount of 0.1-1.0 equivalent based on the 2-deoxy-2,2-difluoro-pentofuranose-l-ulose of Formula (IV').

As the reaction solvent, one or more selected from among ethers such as tetrahydrofuran, diisopropylether, methyl t-butylether and dioxane, nitriles such as acetonitrile, esters such as ethylacetate, isopropylacetate and n-butylacetate, chlorohydrocarbons such as dichloromethane, chloroform and 1,2-dichloroethane, amides such as dimethylacetamide and dimethylformamide, and basic solvents such as pyridine, can be used. Preferably, pyridine is used.

The reaction is carried out by adding 2-naphthoyl halide to the compound of Formula (IV') under ice cooling, stirring the mixture for 30 minutes, and then allowing the stirred solution to react at a temperature between room temperature and the boiling point of the reaction solvent for 1-5 hours.

The D-erythro-2-deoxy-2,2-difluoro-pentofuranose-l-ulose-3,5-di(naphthalene-2-carboxylate) derivatives of Formula (I), resulting from the above-described reaction, can be recovered by concentrating the product under reduced pressure to remove the solvent, extracting the residue the addition of dichloromethane and hydrochloric acid aqueous solution, concentrating the extract, crystallizing the resulting oily residue by the sequential addition of dichloromethane and isopropanol, and washing the crystallized product with cooled isopropanol.

As described above, the present invention provides the novel preparation method, which can easily prepare the erythro compound of Formula (I) by allowing the 2-deoxy-2,2-difluoro-pentofuranose-1-ulose of Formula (IV') to react with 2-naphthoyl halide derivatives.

In the D-erythro-2-deoxy-2,2-difluoro-pentofuranose-1-ulose-3,5-di(naphthalene-carboxylate) derivatives of Formula (I), which are provided according to the present invention as described above, the naphthalene-2-carboxylate derivatives introduced as the hydroxy protecting groups have high crystallinity. Due to this high crystallinity, the desired erythro enantiomer can be easily separated in high yield from the compounds of Formula (I), and thus a process for the mass production of gemcitabine represented by Formula (II) can be more easily performed.

### Example: Preparation of D-erythro-2-deoxy-2,2-difluoro-pentofuranose-l-ulose-3,5-di(naphthalene-2-carboxylate)

4 g (23.8 mmol) of 2-deoxy-2,2-difluoro-pentofuranose-1-ulose (3:1 mixture of erythro enantiomer and threo enantiomer) was dissolved in 40 ml of pyridine, and then 8.49 g (79.2 mmol) of 2,6-lutidine and 1.65 g (13.5 mmol) of 4-dimethylaminopyridine were added thereto. Then, the mixture was stirred. 9.07 g (53.6 mmol) of 2-naphthoyl chloride was dissolved in 15 ml of dichloromethane, and the solution was slowly added to the stirred mixture under ice cooling, followed by stirring for 30 minutes. The reaction solution was slowly heated to reflux for 1 hour, and then the solvent was removed under reduced pressure.

The resulting residue was dissolved in 50 ml of dichloromethane, and then 40 ml of 1N hydrochloric acid was added thereto under ice cooling, followed by stirring. The stirred solution was extracted with dichloromethane and dried with anhydrous magnesium sulfate. The organic layer was concentrated under reduced pressure, and the resulting oil was dissolved in 5 ml of dichloromethane and crystallized by slow addition of 50 ml of isopropanol. The resulting mixture was left to stand at 5 °C for 12 hours, and the solid product was filtered. The filtrate was washed with cooled isopropanol and then dried, thus obtaining 4.08 g (36.0% yield) of pure D-erythro-2-deoxy-2,2-difluoro- pentofuranose-1-ulose-3,5-di(naphthalene-2-carboxylate).

¹H NMR (DMSO-d₆, 300MHz) δ= 4.80-4.87 (m, 2H), 5.50 (q. 1H), 6.18 (m, 1H), 7.52-7.3 (m, 4H), 7.94-8.14 (m, 8H), 8.59 (s, 1H) , 8.74 (s, 1H).

### [Industrial Applicability]

As described above, the present invention provides D-erythro-2-deoxy-2,2-difluoro-pentofuranose-1-ulose-3,5-di(naphthalene-2-carboxylate) derivatives of Formula (I), which are intermediates useful for the preparation of gemcitabine represented by Formula (II) and are prepared by allowing 2-deoxy-2,2-difluoro-pentofuranose-l-ulose of Formula (IV') to react with 2-napthoyl halide derivatives in a reaction solvent in the presence of 2,6-lutidine and 4-dimethylaminopyridine, as well as a preparation method thereof.

In the prior art relating to introducing a protecting group into 2-deoxy-2,2-difluoro-pentofuranose-l-ulose of Formula (IV'), there is a problem in that, due to the low crystallinity of the introduced protecting group, the desired erythro enantiomer is obtained in a very low yield, or a very complicated reaction process should be used to separate an erythro enantiomer from 2,2-difluoro-3-hydroxy-3-(2,2-dimethyl-[1,3]dioxolan-4-yl)propionate represented by Formula (III). However, the present invention has an advantage in that, due to the introduction of the novel protecting group having high crystallinity, the desired erythro enantiomer can be easily prepared in a greatly improved yield.

## Claims

1. D-erythro-2-deoxy-2,2-difluoro-pentofuranose-l-ulose-3,5-di(naphthalene-2-carboxylate) derivatives represented by Formula (I): 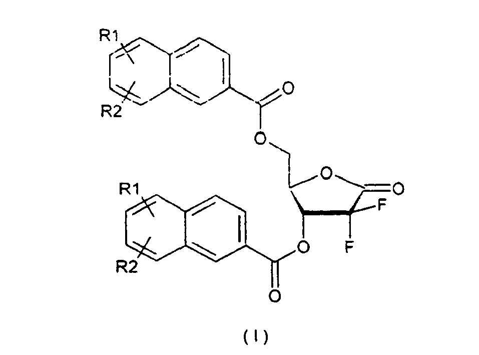 wherein R1 and R2 are each independently hydrogen, methyl, chloro, fluoro, bromo, iodo, methoxy, ethoxy or nitro.

2. The D-erythro-2-deoxy-2,2-difluoro-pentofuranose-l-ulose-3,5-di(naphthalene-2-carboxylate) derivatives of Claim 1, wherein R1 and R2 are each hydrogen and which are represented by Formula (I'):

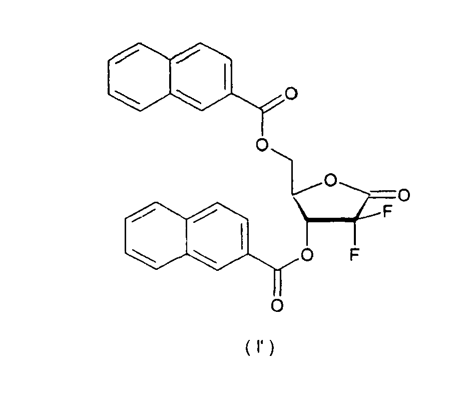

3. A method for preparing D-erythro-2-deoxy-2,2-difluoro-pentofuranose-1-ulose-3,5-di(naphthalene-2-carboxylate) derivatives represented by Formula (I), the method comprising allowing 2-deoxy-2,2-difluoro-pentofuranose-l-ulose of Formula (IV') to react with 2-naphthoyl halide derivatives of Formula (XI) in a reaction solvent in the presence of 2,6-lutidine and 4-dimethylaminopyridine: 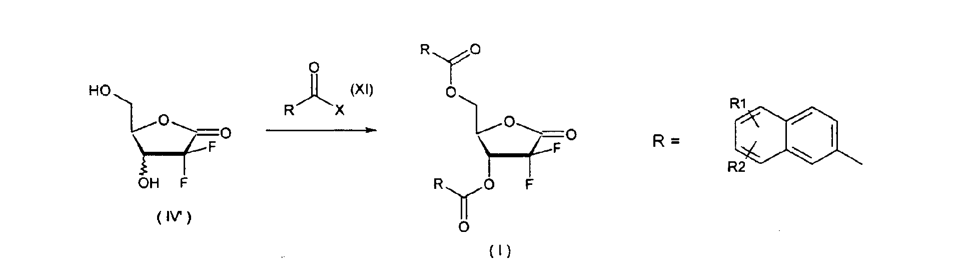 wherein R1 and R2 are each independently hydrogen, methyl, chloro, fluoro, bromo, iodo, methoxy, ethoxy or nitro, and X is chloride or bromide.

4. The method of Claim 3, wherein R1 and R2 are each hydrogen.

5. The method of Claim 3, wherein the reaction solvent is selected from among ethers, including tetrahydrofuran, diisopropylether, methyl t-butylether and dioxane, nitriles, including acetonitrile, esters, including ethylacetate, isopropylacetate and n-butylacetate, chlorohydrocarbons, including dichloromethane, chloroform and 1,2-dichloromethane, amides, including dimethylacetamide and dimethylformamide, and basic solvents, including pyridine.

## Patentansprüche

1. 2-Deoxy-2,2-difluor-D-erythro-pentofuranos-1-ulose-3,5-di(naphthalin-2-carboxylat)derivate der Formel (1): 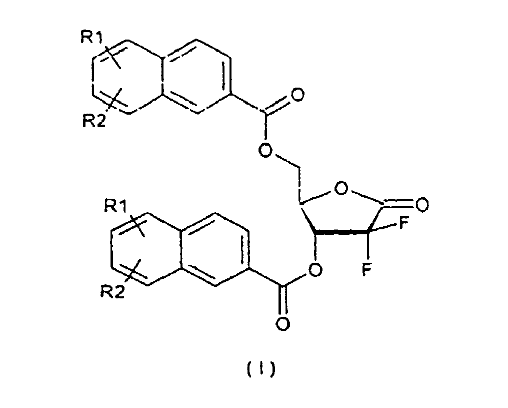 wobei R1 und R2 jeweils unabhängig voneinander Wasserstoff, Methyl, Chlor, Fluor, Brom, Iod, Methoxy, Ethoxy oder Nitro sind.

2. 2-Deoxy-2,2-difluor-D-erythro-pentofuranos-1-ulose-3,5-di(naphthalin-2-carboxylat)derivate nach Anspruch 1, wobei R1 und R2 jeweils Wasserstoff sind und die durch die Formel (1') wiedergegeben werden:

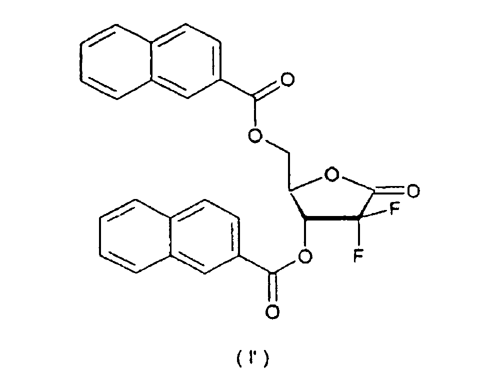

3. Verfahren zur Herstellung von 2-Deoxy-2,2-difluor-D-erythro-pentofuranos-1-ulose-3,5-di(naphthalin-2-carboxylat)derivaten der Formel (I), wobei das Verfahren das Umsetzenlassen von 2-Deoxy-2,2-difluor-pentofuranos-1-ulose der Formel (IV') mit 2-Naphthoylhalogenidderivaten der Formel (XI) in einem Reaktionslösungsmittel in Gegenwart von 2,6-Dimethylpyridin und 4-Dimethylaminopyridin umfasst: 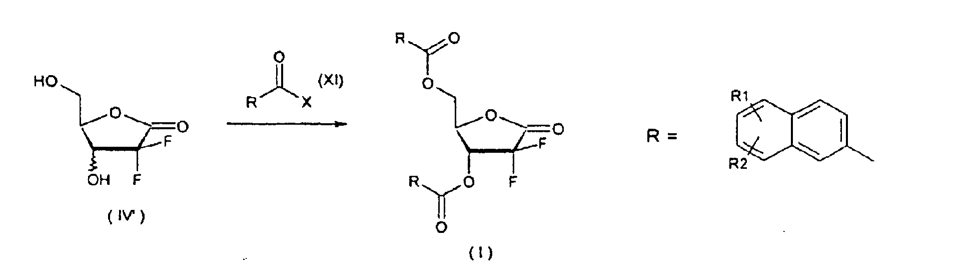 wobei R1 und R2 jeweils unabhängig voneinander Wasserstoff, Methyl, Chlor, Fluor, Brom, Iod, Methoxy, Ethoxy oder Nitro sind und X Chlorid oder Bromid ist.

4. Verfahren nach Anspruch 3, wobei R1 und R2 jeweils Wasserstoff sind.

5. Verfahren nach Anspruch 3, wobei das Reaktionslösungsmittel ausgewählt ist aus Ethern, wie Tetrahydrofuran, Düsopropylether, Methyl-t-butylether und Dioxan; Nitrilen, wie Acetonitril; Estern, wie Ethylacetat, Isopropylacetat und n-Butylacetat; Chlorkohlenwasserstoffen, wie Dichlormethan, Chloroform und 1,2-Dichlormethan; Amiden, wie Dimethylacetamid und Dimethylformamid; und basischen Lösungsmitteln, wie Pyridin.

## Revendications

1. Dérivés de D-érythro-2-désoxy-2,2-difluoro-pentofuranose-1-ulose-3,5-di(naphtalène-2-carboxylate) représentés par la Formule (I) : 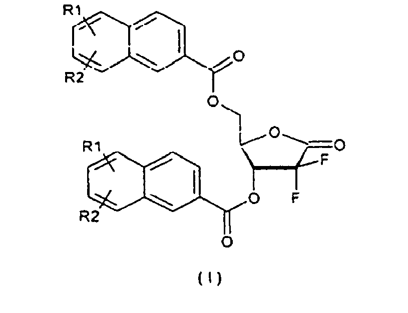 dans laquelle R1 et R2 sont chacun indépendamment hydrogène, méthyle, chloro, fluoro, bromo, iodo, méthoxy, éthoxy ou nitro.

2. Dérivés de D-érythro-2-désoxy-2,2-difluoro-pentofuranose-1-ulose-3,5-di(naphtalène-2-carboxylate) selon la revendication 1, dans lesquels R1 et R2 sont chacun hydrogène et qui sont représentés par la Formule (1') :

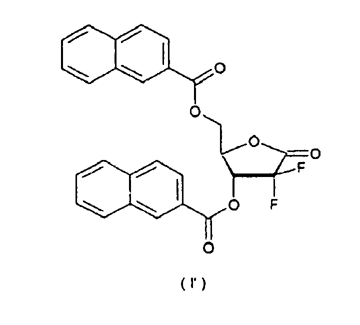

3. Procédé de préparation de dérivés de D-érythro-2-désoxy-2,2-difluoro-pentofuranose-1-ulose-3,5-di(naphtalène-2-carboxylate) représentés par la Formule (I), le procédé comprenant le fait de laisser réagir du 2-désoxy-2,2-difluoro-pentofuranose-1-ulose de Formule (IV') avec des dérivés d'halogénure de 2-naphtoyle de Formule (XI) dans un solvant réactionnel en présence de 2,6-lutidine et de 4-diméthylaminopyridine : 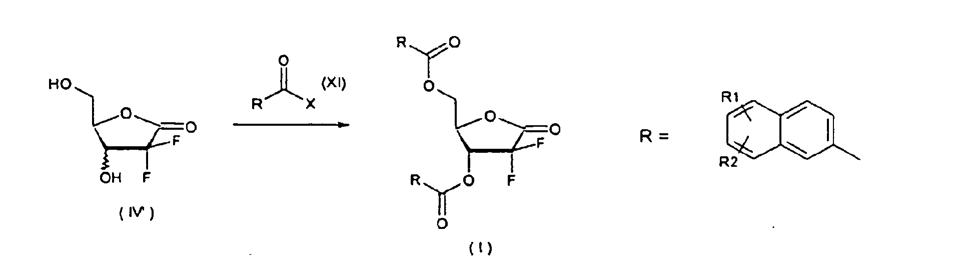 dans laquelle R1 et R2 sont chacun indépendamment hydrogène, méthyle, chloro, fluoro, bromo, iodo, méthoxy, éthoxy ou nitro, et X est chlorure ou bromure.

4. Procédé selon la revendication 3, dans lequel R1 et R2 sont chacun hydrogène.

5. Procédé selon la revendication 3, dans lequel le solvant réactionnel est sélectionné parmi des éthers, y compris le tétrahydrofurane, le diisopropyléther, le t-butyléther méthylique et le dioxane, des nitriles, y compris l'acétonitrile, des esters, y compris l'éthylacétate, l'isopropylacétate et le n-butylacétate, des chlorohydrocarbures, y compris le dichlorométhane, le chloroforme et le 1,2-dichlorométhane, des amides, y compris le diméthylacétamide et le diméthylformamide, et des solvants basiques, y compris la pyridine.
